# EUROPEAN PATENT APPLICATION

(11) **EP 4 431 108 A1**
(43) Date of publication of application: **18.09.2024**
(21) Application number: 22891610.2
(22) Date of filing: 06.09.2022
(51) Int. Cl.: A61K 38/08, A61K 38/10, A61K 47/64, A61K 47/68, A61P 25/32, A61P 25/36, C07K 14/705, C12N 15/12

(54) **USE OF POLYPEPTIDE IN RESISTANCE TO ADDICTION AND RELAPSE THEREOF, AND COMPLEX AND POLYPEPTIDE**

(30) Priority: 09.11.2021 CN 202111321033
(71) Applicant: Shenzhen Chenyang Biological Technology Co., Ltd, Shenzhen, Guangdong 518000 (CN)
(72) Inventor: TAN, Zhen, Shenzhen, Guangdong 518000 (CN); LI, Tao, Shenzhen, Guangdong 518000 (CN); LI, Shupeng, Shenzhen, Guangdong 518000 (CN)
(74) Representative: Cabinet Becker et Associés
(86) International application number: PCT/CN2022/117272
(87) International publication number: WO 2023/082805

(57) **Abstract**

The present invention relates to the use of polypeptide in resistance to addiction and relapse thereof. Provided is the use of a polypeptide in the preparation of a drug for treating and/or preventing substance addiction or relapse thereof. The polypeptide consists of at least 11 continuous amino acid residues in a sequence as shown in SEQ ID NO: 1, and contains a sequence as shown in SEQ ID NO: 2. The present invention also relates to the uses of the polypeptide, a nucleic acid molecule, an expression vector and a host cell, and a complex containing the polypeptide.

## Description

### Field of the Invention

The present invention relates to the field of substance addiction, in particular to the use of a polypeptide in preventing and treating addiction or the relapse of addiction.

### Background of the Invention

The World Health Organization (WHO) defines substance addiction as the repeated use of a substance despite knowing and experiencing the harmful effects thereof. Substance addiction is a chronic recurrent disease characterized by uncontrolled use of a substance, compulsive search and craving for a substance, persistent use regardless of adverse consequences and physical and/or psychological dependence on a substance. Generally speaking, substance addiction follows the process of tolerance, withdrawal, compulsive drug use behavior, drug seeking behavior, addiction behavior implementation and relapse.

Substance addiction has become a medical and social problem of global concern, which has great social and economic impacts on both the addicts and the society. For example, substance addiction is often closely related to violent crimes and the spread of infectious diseases. Another example is that substance addiction may cause partial or total loss of the addicts' labor force, thereby bringing negative impacts on individuals, families and the society. Specifically, as one of the most commonly abused substances in the world, alcoholism leads to serious liver and cardiovascular diseases and is prone to severe mental disorders, social problems and adverse consequences, including family disintegration, tragic accidents and reduced working efficiency.

Some withdrawal medicaments are often used in drug detoxification or withdrawal treatment for addicted subjects, but these medicaments may still have limitations.

For example, in the case of addiction caused by opioids, the methods commonly used include: (i) substitution therapy, and substitution by methadone is currently the most commonly used method for drug detoxification; (ii) Opioid receptor antagonist, naltrexone or naloxone for precipitated withdrawal; (c) Non-opioid treatment, using clonidine and lofexidine to suppress the withdrawal symptoms. Although these methods may keep an addicted subject from showing physical discomfort (such as chills, vomiting, tremors, etc.) after stopping using an addictive substance, studies have shown that these medicaments cannot reduce the mental dependence (or psychological dependence) of the addicted subject.

For another example, the potential therapeutic effects of different types of medicaments (such as naltrexone, acamprosate, ondansetron, disulfiram, Y-hydroxybutyric acid (GHB) and topiramate) on alcohol addiction have been tested. Several of these pharmaceutical therapeutic agents such as naltrexone, acamprosate and disulfiram have been verified to have a certain effect and have been approved for the treatment of alcoholism. Among these medicaments, naltrexone is currently considered as a choice with better pharmacology. However, although there are certain promising results, none of these medicaments (including naltrexone) are effective enough in alcoholism and the prognosis is still poor.

Therefore, in the field of anti-substance addiction, there is a strong demand to develop a safe and effective medicament to provide more plentiful and better treatment or prevention strategies for substance addiction and the relapse thereof.

### Summary of the Invention

In order to achieve the above purpose, the inventors have conducted a large number of studies on the molecular mechanism of addiction, and found that a new polypeptide can destroy the interaction between D2 receptor and NMDA receptor.

Specifically, DA receptor is a kind of receptor located in an organism which acts by means of its corresponding membrane receptor. DA receptors can be divided into five types: D1, D2, D3, D4 and D5. D2 receptor (D2R) is widely expressed in brain.

The NMDA receptor (NMDAR) not only plays an important physiological role in the development of the nervous system, such as regulating the survival of neurons, regulating the development of dendrites and axons of neurons and participating in the formation of synaptic plasticity, but also plays a key role in the formation of neuronal circuits and in the pathogenesis of various neuropsychiatric diseases. Data show that the NMDA receptor is a kind of crucial receptor in the process of learning and memory. NMDAR is mainly composed of three different subunits, which are NR1, NR2 and NR3, respectively. Wherein the NR2 subunit has four different subunits, namely, NR2A, NR2B, NR2C and NR2D.

The inventors have confirmed that the polypeptide of the present invention is effective in treating addiction of addictive substances and inhibiting or treating relapse, thereby completing the present invention.

Accordingly, in a first aspect, the present invention provides a polypeptide consisting of at least 11 consecutive amino acid residues of the sequence as set forth in SEQ ID NO: 1 and containing the sequence as set forth in SEQ ID NO: 2. Also provided is the use of the polypeptide of the present invention in the preparation of a medicament for preventing and/or treating substance addiction.

As used herein, the term "treat" refers to causing a desired or beneficial effect in a patient, which may include reducing the frequency or severity of one or more symptoms of a disease, or hindering or inhibiting the further development of a disease, condition or disorder.

As used herein, the term "prevent" means avoiding or delaying the onset of a disease or avoiding the onset of its clinical or subclinical symptoms.

As used herein, the term "substance addiction" means a chronic recurrent disease characterized by uncontrolled use of a substance, compulsive search and craving for a substance, persistent use regardless of adverse consequences, and physical and/or psychological dependence on a substance. Substance addiction may, for example, be an addiction caused by the substance for the first time, or refer to the formation and/or expression of substance addiction.

In an exemplary embodiment, the polypeptide of the present invention consists of at least 11, at least 12, at least 13, at least 14, or at least 15 consecutive amino acid residues of the sequence as set forth in SEQ ID NO: 1.

In an exemplary embodiment, the sequence of the polypeptide of the present invention consists of positions 1-15, positions 1-14, positions 1-13, positions 2-15, positions 2-14, positions 2-13, positions 3-15, positions 3-14, or positions 3-13 in SEQ ID NO: 1 (KIYIVLRRRRKRVNT).

In a specific embodiment, the sequence of the polypeptide of the present invention is as set forth in SEQ ID NO: 1, SEQ ID NO: 3 or SEQ ID NO: 4.

In some embodiments, the addictive substance may, for example, be selected from the group consisting of morphine, nicotine, alcohol, cocaine, codeine, dihydrocodeine, hydromorphone, oxycodone, methadone, morphine, fentanyl, and pethidine.

In a second aspect, the present invention provides the use of the polypeptide of the present invention in the preparation of a medicament for preventing and/or treating the relapse of substance addiction.

As used herein, "relapse", also known as "drug relapse" where appropriate, refers to the case that an addict resumes using the additive substance which was used before after going through withdrawal. Relapse can be caused by the factors of environment (occasion), addictive substances and/or stress (pressure).

In an exemplary embodiment, the relapse in the present invention is caused by environmental factors.

In an exemplary embodiment, the relapse in the present invention is caused by a factor of addictive substances.

In an exemplary embodiment, the relapse in the present invention is caused by a stress factor.

The present inventors unexpectedly discovered that the polypeptide of the present invention can block the binding of the D2R/NR2B complex and eliminate the formation of the addictive substance.

Furthermore, the polypeptide of the present invention can eliminate the relapse of addiction caused by the factors of environment, addictive substances and stress, and even achieve statistically significant effects under the condition induced by high/low doses of addictive substances, thus having good clinical development value.

In a third aspect, the present invention provides a nucleic acid molecule encoding a polypeptide of the present invention. In addition, also provided is the use of the nucleic acid molecule of the present invention in the preparation of a medicament for treating and/or preventing substance addiction, or the use of the nucleic acid molecule of the present invention in the preparation of a medicament for treating and/or preventing the relapse of substance addiction.

The nucleic acid molecule of the present invention is used to produce the polypeptide of the present invention, and the sequence of the nucleic acid molecule can be appropriately adjusted by those skilled in the art according to the expression system employed.

In an exemplary embodiment, the nucleic acid molecule has a sequence as set forth in SEQ ID NO: 5.

In a fourth aspect, the present invention provides an expression vector comprising the nucleic acid molecule of the present invention. In addition, also provided is the use of the expression vector of the present invention in the preparation of a medicament for treating and/or preventing substance addiction, or the use of the expression vector of the present invention in the preparation of a medicament for treating and/or preventing the relapse of substance addiction.

A nucleic acid sequence of the present invention can be inserted into an expression vector using a variety of known methods. For example, a nucleic acid molecule can be inserted into an appropriate restriction endonuclease site. Standard techniques for cloning, isolation, amplification and purification, and enzymatic reactions involving DNA ligase, DNA polymerase, restriction endonuclease and various separation techniques in operation belong to techniques known and commonly used by those skilled in the art.

In a fifth aspect, the present invention provides a host cell comprising the nucleic acid molecule or expression vector of the present invention. In addition, also provided is the use of the host cell of the present invention in the preparation of a medicament for treating and/or preventing substance addiction, or the use of the host cell of the present invention in the preparation of a medicament for treating and/or preventing the relapse of substance addiction.

The polypeptide of the present invention may be produced using expression vectors and host cells in a variety of expression systems such as prokaryotic and eukaryotic expression systems. Next, the mammalian expression system is illustrated as an instance. The host cell may include COS-7 cell line of monkey kidney fibroblasts and other cell lines capable of expressing compatible vectors, such as C127, 3T3, CHO, Hela and BHK cell lines. A mammalian expression vector should comprise a replication origin, a suitable promoter and enhancer, and any necessary ribosome binding site, polyadenylation site, splicing donor and receptor site, transcription termination sequence, and 5' flanking non-transcriptional sequence. DNA sequences derived from, for example, SV40 splicing and polyadenylation sites can be used to provide the desired non-transcriptional genetic elements. An expression vector may be introduced into a host cell by a variety of methods familiar to those skilled in the art, including but not limited to e.g. calcium phosphate transfection, DEAE-glucan mediated transfection or electroporation.

In a sixth aspect, the present invention provides a complex comprising the polypeptide of the present invention and a (transferring) carrier attached thereto for permeating the blood-brain barrier.

In an exemplary embodiment, the carrier used to permeate the blood-brain barrier may be one or more of HIV-1 Tat protein, insulin, cationized albumin, monoclonal antibody against rat transferrin receptor (OX26), mouse-derived monoclonal antibody against human insulin receptor (HIRMAb), Penetratin, transduction domain of Tat protein, Pep-1 peptide, S413-PV, Magainin 2 and Buforin 2. For example, TAT transduction domain, whose amino acids are YGRKKRRQRRR (as set forth in SEQ ID NO: 6), can transduce across the membrane into a cell.

The polypeptide of the present invention can be linked to a carrier for permeating the blood-brain barrier by appropriate linking techniques. Exemplary linking techniques may be avidin-biotin techniques, polyethylene glycol (PEG)-based space arm techniques, fusion protein techniques and the like. For example, in the case of using the transduction domain of HIV-1 Tat protein as a carrier for permeating the blood-brain protein as a carrier for permeating the blood-brain barrier, the polypeptide of the present invention can be directly linked to the transduction domain of Tat protein by fusion protein techniques.

In some embodiments, where fusion protein techniques are used, the polypeptide of the present invention can also be linked to a carrier for permeating the blood-brain barrier using a linker. Exemplary linkers may be flexible linkers with glycine, such as G, GSG, GSGGSG, GSGGSGG, GSGGSGGG, GGGGSGGG, GGGGS and SGG, etc.

In a seventh aspect, the present invention provides a method for treating or preventing substance addiction in a subject, comprising:
administering an effective amount of the polypeptide or complex of the present invention to the subject.

In addition, the present invention provides a method for preventing relapse of substance addiction in a subject, comprising:
administering an effective amount of the polypeptide or complex of the present invention to the subject.

The polypeptide or complex reduces the interaction between D2R and NR2B, thereby achieving the above method.

The term "effective amount" or "therapeutically effective amount" refers to an amount of an active agent sufficient to induce a desired biological result. The result may be a reduction in the signs, symptoms or causes of a disease, or any other desired change of a biological system. The term "therapeutically effective amount" is used herein to mean any amount of a formulation that causes a substantial improvement in the condition of the disease when repeatedly administered to the affected area over a period of time. This amount will vary depending on the condition being treated, the stage of progression of the condition, and the type and concentration of the formulation used. Those skilled in the art can determine the appropriate amount by routine experiments.

"Subject", "individual" or "patient" are used interchangeably herein and refer to a vertebrate, preferably a mammal, more preferably a human. Mammals include, but are not limited to, rats, monkeys, humans, farm animals, and pets. Tissues, cells and their descendants of biological entities obtained or cultured in vitro are also included.

In an eighth aspect, the present invention provides a polypeptide or complex of the present invention, which is used as a medicament.

In some embodiments, being used as a medicament means being used to treat or prevent substance addiction.

In some embodiments, being used as a medicament means being used to prevent the relapse of substance addiction.

### Brief Description of the Drawings

Fig. 1 shows the results of co-immunoprecipitation of hippocampus tissue from mice;
Fig. 2 shows the positions of the fragments involved in Example 3 on the D2R;
Fig. 3 shows the results of GST-pulldown experiments for each fragment of Example 3;
Fig. 4 shows the co-immunoprecipitation results of hippocampus tissue from KT polypeptide-treated CPP mice and BS mice;
Fig. 5 shows the experimental scheme design of Example 5;
Fig. 6 shows the CPP score results of Example 5;
Fig. 7 shows the experimental scheme design of Example 6;
Fig. 8 shows the CPP score results of Example 6; A: Measurement results of each group on Day 10; B: Comparison results of TAT-D2R-KT Group on Day 11 versus Day 10;
Fig. 9 shows the experimental scheme design of Example 7;
Fig. 10 shows the CPP measurement result on Day 10 of Example 7;
Fig. 11 shows the CPP measurement results of Test 1 of Example 7;
Fig. 12 shows the CPP measurement results of Test 2 of Example 7;
Fig. 13 shows the CPP measurement results of Test 3 of Example 7;
Fig. 14 shows the CPP measurement results of Test 4 of Example 7;
Fig. 15 shows the experimental scheme design of Example 8;
Fig. 16 shows BS measurement results of Test 1 of Example 8; A: BS moving distance results of Test 1; B: BS moving distance results of Test 2; C: BS moving distance results of Test 3;
Fig. 17 shows the effect of different polypeptides prepared in Example 9 on morphine-induced CPP in mice, wherein n=12; ##: P<0.01, relative to normal saline group; **: P<0.01, relative to morphine group;
Fig. 18 shows the negative effect of different polypeptides prepared in Example 9 on cocaine-induced behavioral sensitization in mice, wherein n=10; *: P<0.05, **: P<0.01, relative to veh group;
Fig. 19 shows the negative effect of different polypeptides prepared in Example 9 on alcohol-induced behavioral sensitization in mice, wherein n=10; ##: P<0.01, relative to normal saline group; **: P<0.01, relative to alcohol group.

### Detailed Description of the Invention

At present, how to eliminate substance addiction or prevent its relapse so as to avoid the great burden thereof on individuals, families and the society has become a medical and social problem of global concern. Wherein interestingly, Liu et al. (Modulation of D2R-NR2B Interactions in Response to Cocaine, Neuron 52, 897-909, Decree 7, 2006) reported that dopamine stimulation brought by cocaine enhanced the formation of hetero-receptor-complex between D2R and the NR2B subunit of NMDA receptor in neostriatum in vivo; by studying the interaction between D2R and NR2B, it is found that the region responsible for the formation of D2R-NR2B complex in D2R is located in IL3; further competition experiments showed that the motif containing ten residues (TKRSSRAFRA, T225-A234) in IL3 is the key to prevent the binding of D2R to NR2B.

It should be noted that Liu et al. tested a variety of polypeptides of IL3 from D2R, and the results showed that (see Figs. 3M and 3N in said literature): only in the case of containing the above motif T225-A234, polypeptides (P1, P2, P3 and P5) successfully prevented the binding of D2R to NR2B; while in the absence of this motif, the polypeptide (P4, I210-K226) had no preventing effect; at the same time, the control peptide P6 obtained by scrambling the sequence of peptide P5 which contains this motif also could not prevent the binding of D2R to NR2B. Therefore, Liu et al. believe that the binding is sequence-specific, and TKRSSRAFRA is a potential binding motif.

However, the present inventors unexpectedly found in their studies that, unlike the report of Liu et al., the polypeptide K211-T225 in the IL3 region of D2R (which has a deletion of one residue at the N-terminal and one residue at the C-terminal as compared to the polypeptide P4, which is considered unable to prevent the binding of D2R to NR2B; and does not contain the key motif TKRSSRAFRA) successfully reduced the interaction between D2R and NR2B.

On the basis of further research, the present inventors conclusively demonstrated that the polypeptide K211-T225 in the IL3 region of D2R and its variant with a deletion at N-terminal and C-terminal can reverse the addiction behavior change in an addiction model.

Throughout the specification, terms used herein shall be understood to have the meanings commonly used in the art unless otherwise specifically stated. Accordingly, unless otherwise defined, all technical and scientific terms used herein have the same meaning as generally understood by those skilled in the art to which the present invention belongs. In case of any contradiction, the present application shall take precedence.

Embodiments of the present invention will be described in detail below in connection with the Examples, and the advantages and various effects of the present invention will be more clearly presented therefrom. It shall be understood by those skilled in the art that these specific embodiments and Examples are intended to illustrate but not limit the present invention.

If the specific condition is not specified in an Example, a conventional condition or the condition recommended by the manufacturer shall be followed. If the manufacturer of a reagent or instrument used is not indicated, it is a commercially available conventional product.

Unless otherwise stated, the experimental animals used in the Examples were adult C57/BL6J male mice (8 weeks old, weight 25-30g) purchased from Guangzhou Medical Laboratory Animal Center, China. The mice were kept under 12-hour illumination/12-hour dark cycle, with free access to food and water throughout the process. Before the experimental operation, all mice were adapted in the animal room for one week.

In the Examples, Imaged and Image Lab software (ImageJ1.30) were used to analyze the bands and morphological data, and GraphPadPrism8 software was used for statistical analysis. Sample amounts were selected as described earlier (Arifin and Zahiruddin, 2017; Mead et al., 2012). All experiments were repeated three times independently. Randomized method/blind method was not used. Data are presented as mean ± SEM. One-way or two-way ANOVA was used in different groups, followed by postshoc Tukey multiple comparison test. P<0.05 is considered statistically significant. Unless otherwise stated, *: p<0.05, **: p<0.001, ***: p<0.0001, and ****: p<0.000001.

### Example 1. Confirmation of formation of D2R/NR2B complex in vivo

### Immunoprecipitation operation:

Co-immunoprecipitation was performed using a protein sample (100-500 µg protein) from hippocampus tissue. NR2B and D1R or D2R were precipitated using an anti-NR2B and D2R, D2R or NR2B and 25 µl protein A/G + agarose bead slurry (SantaCruz Biotechnology, sc-2001), respectively.

The protein was then isolated on 8% SDS-PAGE gel and detected with a mouse antibody against NR2B and D2R. HRP-coupled secondary antibody and enhanced chemiluminescence method were used to detect the protein. Density analysis of bands was performed using image laboratory software.

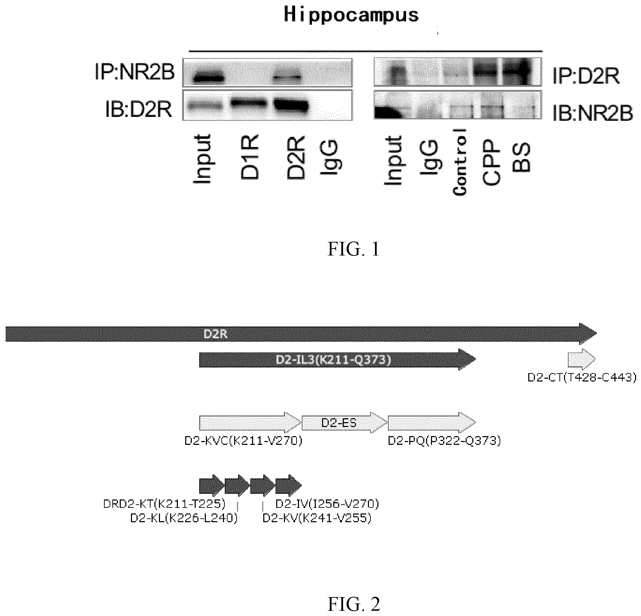

In order to assess whether D2R and NR2B can form a protein complex, the above co-immunoprecipitation method was firstly performed, and possible interaction between D2R and NR2B in normal mouse hippocampus was examined. The results were shown in Fig. 1.

As can be seen from Fig. 1, NR2B was successfully precipitated from hippocampus protein mixture by a D2R antibody, while NR2B was not precipitated by a D1R antibody, indicating D2R/NR2B protein coupling.

### Example 2. Verification of pathological significance of the complex

Next, in order to determine whether the interaction state of D2R and NR2B is related to substance addiction, an immunoprecipitation experiment was performed on samples of hippocampus tissue from mice with morphine-induced place preference (CPP) and behavioral sensitization (BS) according to the method in Example 1, and the results were shown in Fig. 1.

As can be seen from Fig. 1, D2R and NR2B coupling increased significantly in hippocampus tissue from both CPP and BS mice, which confirmed that the formation of D2R/NR2B complex may play an etiological role in substance addiction.

### Example 3. Search for complex interbinding site

In order to search for the physical interaction site between D2R and NR2B, cDNA fragments of the CT region of D2R (T428-C443) and the third intracellular loop (IL3) region of D2R (K211-Q373) were first obtained via the D2R full-length cDNA clone (Genbank Accession No.: M29066.1). These fragments were subcloned to the BamH1/EcoR1 or BamH1/Xho1 site of a pGEX-4T-3 plasmid (YouBio, Item No: VT1255). Initiation methionine residue and termination codon were added as appropriate. All constructs were resequenced to confirm that splice fusion was achieved appropriately. The GST fusion protein containing the IL3 region of D2R (GST-D2R-IL3) and the GST fusion protein containing the CT region of D2R (GST-D2R-CT) were obtained by using *E.coli* BL21 viable cells (AlpalifeBio, Item No: KTSM104L) for expression and purifying them from bacterial lysate, wherein the specific positions of the coding sequences of IL3 region and CT region on D2R were shown in Fig. 2.

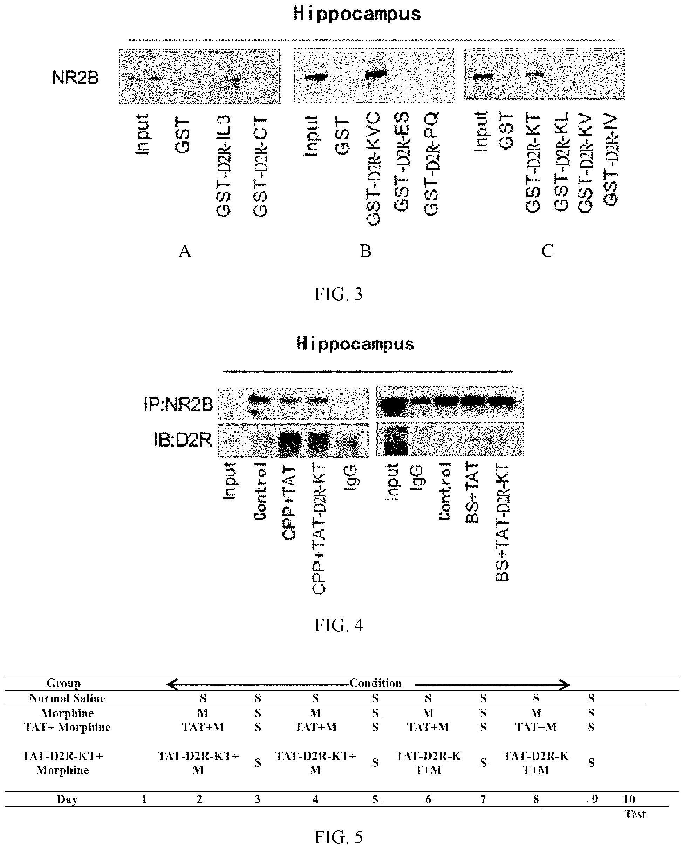

500 µg of dissolved hippocampus extract was diluted with 1 × PBS/0.1% Triton X-100 and incubated overnight with 20 µl protein GST resin (TransGen Biotech, Item No: N21220, Beijing, China) saturated with GST protein only or with the prepared 15 µg GST fusion protein at 4 °C. Beads were washed 1-8 times with 1 × PBS/0.1% Triton X-100. Binding proteins were eluted with 2 × loading buffer, separated by SDS-PAGE, and subjected to Western blot with respective antibodies thereof, and the results were shown in A of Fig. 3.

As can be seen from A of Fig. 3, NR2B was precipitated with affinity by the IL3 region of D2R, indicating that the IL3 region of D2R participates in the interaction.

Next, in order to further explore the interaction sequence/site between D2R and NR2B, the IL3 region was divided into KVC (K211-V270) region of D2R, ES (E271-S321) region of D2R and PQ (P322-Q373) region of D2R. The specific positions of these regions on D2R were shown in Fig. 2.

According to the method in the present Example, a pulldown analysis was performed using a GST fusion protein containing the KVC region of D2R (GST-D2R-KVC), a GST fusion protein containing the ES region of D2R (GST-D2R-ES), and a GST fusion protein containing the PQ region of D2R (GST-D2R-PQ), and the results of Western blot were shown in B of Fig. 3.

As can be seen from B of Fig. 3, NR2B was precipitated with affinity by the KVC region of D2R, indicating that the KVC region of D2R participates in the interaction.

Further, the KVC region was divided into KT (K211-T225) region of D2R, KL (K226-L240) region of D2R, KV (K241-V255) region of D2R, and IV (I256-V270) region of D2R. The specific positions of these regions on D2R were shown in Fig. 2.

According to the method in the present Example, a pulldown analysis was performed using a GST fusion protein containing the KT region of D2R (GST-D2R-KT), the GST fusion protein containing the KL region of D2R (GST-D2R-KL), a GST fusion protein containing the KV region of D2R (GST-D2R-KV), and a GST fusion protein containing the IV region of D2R (GST-D2R-IV), and the results of Western blot were shown in C of Fig. 3.

Surprisingly, as shown by C of Fig. 3, NR2B could not be precipitated by the KL region of D2R (K226-L240), while NR2B was precipitated with affinity by the KT region of D2R (K211-T225, hereinafter referred to as KT polypeptide).

### Example 4. Polypeptide inhibits complex coupling in vivo

In order to verify the effect of KT polypeptide of D2R on the D2R/NR2B complex in vivo, the C terminal of the region (K211-T225) was fused to the N terminal of transduction domain of HIV-1-type Tat protein (as set forth in SEQ ID NO: 6, hereinafter referred to as TAT) to obtain a fusion protein which can permeate blood-brain barrier, named TAT-D2R-KT.

CPP mice and BS mice were treated with TAT-D2R-KT respectively, and mice treated with TAT only were used as control (all treated by single intraperitoneal administration, 10ml/kg body weight, medicament concentration 3nmol/g). After 1 hour of treatment, analysis was performed according to the co-immunoprecipitation method in Example 1, and the results were shown in Fig. 4.

As can be seen from Fig. 4, the KT polypeptide of the present invention competitively disrupted the D2R/NR2B interaction in the hippocampus tissue of CPP mice and BS mice.

### Example 5. Polypeptide interfered with morphine-induced CPP formation

In order to evaluate whether the polypeptide of the present invention affects morphine addiction, CPP scores were measured with TAT-D2R-KT and TAT at the formation stage of morphine-induced CPP.

CPP apparatus and testing system are as follows:
The CPP equipment used in the experiment has two equal-volume chambers (15 cm × 15 cm × 37 cm) with a movable door in the middle. Mice could freely shuttle between chambers during testing, or were limited to one side of the chamber during training. The interior of one chamber is black and the bottom is striped, while the other chamber is white and the bottom is latticed. All behavioral tests, including distance and time of activity, were recorded by a camera and calculated using the SMART Video Tracking System (Version 2.5; Panlab Technology for Biosearch, Spain).

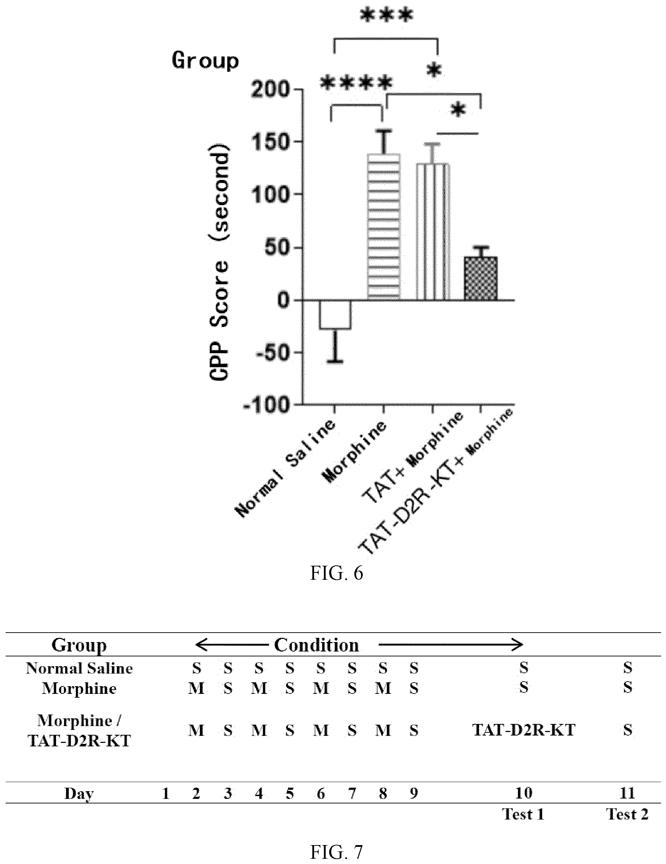

The experimental design was shown in Fig. 5. On Day 1, mice were allowed to explore two chambers freely for 15min without any treatment (before testing). On Day 2, 4, 6 and 8, control group (saline group) and model group (morphine group) received saline (10mL/kg) and morphine (10mg/kg), respectively. TAT group (3nmol/g) and TAT-D2R-KT group (3nmol/g) were injected (intraperitoneally, 10ml/kg body weight) 1 hour before morphine injection. After injection, all mice were trained in the white chamber for 40min. On Day 3, 5, 7 and 9, all mice were given normal saline, and then immediately placed in the black chamber for 40min; on Day 10, after all mice explored freely for 15min, the test ended, and the CPP scores (moving duration of mice) were shown in Fig. 6.

As can be seen from Fig. 6, TAT-D2R-KT treatment significantly reduced CPP score compared with TAT-treated subjects, indicating that TAT-D2R-KT can interfere with morphine-induced CPP formation.

### Example 6. Effect of polypeptide on the expression stage of morphine-induced CPP

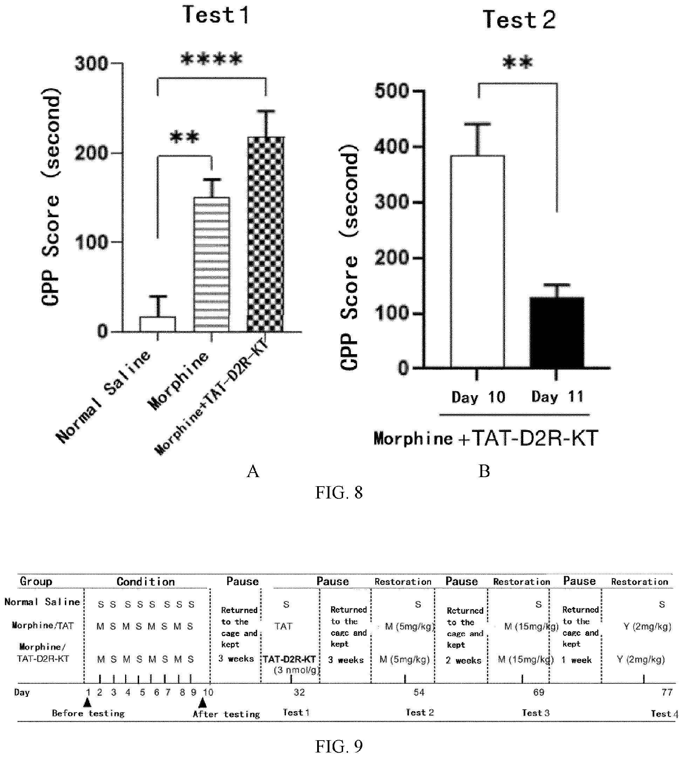

To further evaluate the specific effect of the polypeptide of the present invention on the expression stage of morphine-induced CPP, a test was performed using the CPP apparatus in Example 5 and according to the experimental design in Fig. 7, wherein model group (morphine group) was injected with normal saline on Day 10, while in TAT-D2R-KT group (morphine + TAT-D2R-KT), CPP mice were treated with TAT-D2R-KT (single dose) on Day 10, and the CPP scores were measured 1 hour later. The experimental results were shown in A of Fig. 8.

As shown by A of Fig. 8, treatment with TAT-D2R-KT on Day 10 did not significantly reduce the CPP score.

Nevertheless, by repeating CPP test on Day 11, we found that the CPP score of TAT-D2R-KT group decreased significantly on Day 11 compared with that on Day 10 (B of Fig. 8), which indicated that TAT-D2R-KT might eliminate the related memory when morphine addiction memory reappeared on Day 10.

### Example 7. Polypeptide eliminated morphine-induced drug relapse

In order to verify that the polypeptide of the present invention can eliminate the related memory when addiction memory reappears and test its long-term effect on the drug relapse of morphine CPP, we designed the natural withdrawal and drug relapse experiment of CPP mice as shown in Fig. 9, and carried out a number of tests, in which the normal saline group served as the control.

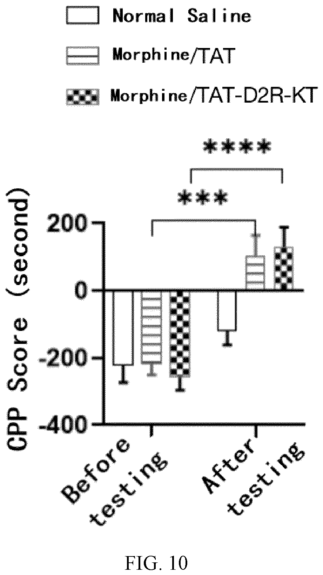

According to the conditions shown in Fig. 9, morphine successfully induced CPP in mice on Day 10, and the results were shown in Fig. 10.

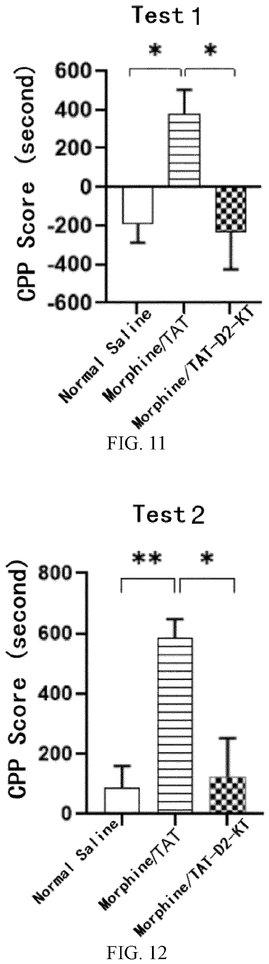

The mice were then returned to the cage and kept for three weeks. On Day 32, mice in TAT group and TAT-D2R-KT group were treated with TAT and TAT-D2R-KT (3nmol/g) respectively. After 1 hour, the mice were free to explore for 15min for Test 1, and the results were shown in Figure 11.

As can be seen from Fig. 11, the CPP score of mice in TAT-D2R-KT group was significantly lower than that of the TAT group, indicating that the polypeptide of the present invention can eliminate drug relapse induced by the environment.

In order to further explore the possible effect of the polypeptide on drug relapse induced by addictive drugs, after Test 1, each group continued to be returned to the cage and kept for three weeks, and morphine (5mg/kg) was injected to TAT group and TAT-D2R-KT group on Day 54. All mice were immediately put into the experimental chamber to explore freely for 15min for Test 2, and the results were shown in Fig. 12.

As can be seen from Fig. 12, the CPP score of mice in TAT-D2R-KT group was again significantly lower than that of the TAT group.

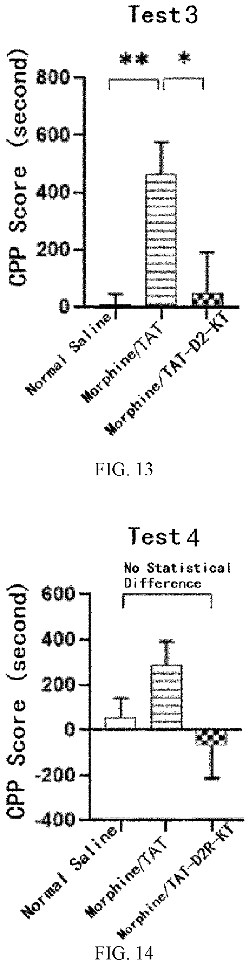

To rule out the dose effect, each group was returned to the cage and kept for two weeks after Test 2, and mice in TAT and TAT-D2R-KT groups were injected with a higher dose of morphine (15mg/kg) on Day 69. Then all mice were free to explore for 15 minutes for Test 3, the results were shown in Fig. 13.

Surprisingly, a higher dose of morphine still could not reverse the significant reduction of the CPP score caused by TAT-D2R-KT, which indicated that TAT-D2R-KT could eliminate drug relapse induced by a low/high dose of addictive substance.

To further explore the effect of TAT-D2R-KT on pressure-induced drug relapse, each group was returned to the cage and kept for one week after Test 3 and on Day 77 mice in both TAT and TAT-D2R-KT groups were administered yohimbine (α2-adrenergic receptor antagonist, 2mg/kg, purchased from MCE, Item No: 18735) and then tested for 15 minutes after the administration, noted as Test 4, and the results were shown in Fig. 14.

As can be seen from Fig. 14, there was no significant difference in CPP scores between mice in TAT-D2R-KT group and control group, indicating that the polypeptide of the present invention successfully blocks drug relapse induced by stress.

### Example 8. Verification for resistance against addiction-related behaviors using behavioral sensitization model

In order to further verify the effect of the polypeptide of the present invention on morphine addiction-related behaviors, the above results were verified by using a morphine-induced behavioral sensitization mouse (BS) model.

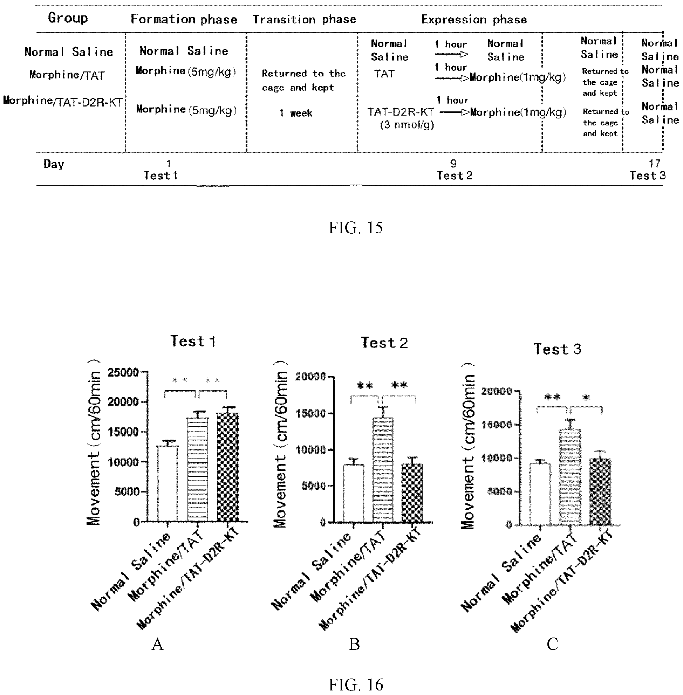

The experiment was conducted according to the scheme shown in Fig. 15. On Day 1, all mice were adapted to the experimental chamber for one hour. Then, mice were given saline (control group) or 5mg/kg morphine (TAT group and TAT-D2R-KT group) and entered into the experimental chamber (with a white surface and flat bottom, 50cm × 50cm × 35cm³ in size), the movement activities were immediately recorded for 60min. The effect of the medicaments was evaluated with the total walking distance of mice as a measure (noted as Test 1), and the results were shown in A of Fig. 16.

All mice were then returned to the cage and kept for 1 week. On Day 9, mice were given TAT or TAT-D2R-KT (3nmol/g) one hour before morphine (1mg/kg) usage, and then all mice entered into the experimental chamber for testing to evaluate the effect of the medicaments (noted as Test 2). The results shown in B of Fig. 16 showed that the 60-minute movement activity of mice in TAT-D2R-KT group decreased significantly.

After Test 2, all mice were returned to the cage and kept for one week (withdrawal), and then re-entered into the experimental chamber for testing (noted as Test 3). The results shown in C of Fig. 16 showed that a significant decrease in movement activity could still be detected in TAT-D2R-KT group after 1 week of withdrawal, which further indicated that morphine-induced drug relapse could be eliminated by the polypeptide.

### Example 9. Design and preparation of polypeptide variants

Based on KT polypeptide (noted as Pep1), variant Pep2 with an N-terminal deletion, variant Pep3 with a C-terminal deletion, variant Pep4 with the mid-segment RRR mutated to GGG, and variant Pep5 with the mid-segment RKR mutated to GGG were designed and synthesized, respectively, as shown in detail in Table 1 below:

**Table 1**

| Name | Sequence | | Mutation type |
|---|---|---|---|
| Pep1 (SEQ ID NO: 1) | | KIYIVLRRRRKRVNT | N.A. |
| Pep2 (SEQ ID NO: 3) | | YIVLRRRRKRVNT | N-terminal truncated |
| Pep3 (SEQ ID NO: 4) | | KIYIVLRRRRKRV | C-terminal truncated |
| Pep4 (SEQ ID NO: 7) | | KIYIVLGGGRKRVNT | Mid-segment RRR substituted |
| Pep5 (SEQ ID NO: 8) | | KIYIVLRRRGGGVNT | Mid-segment RKR substituted |

Then, the C-terminal of Pep1 to Pep5 was fused to the N-terminal of TAT respectively to obtain fusion peptides which could penetrate the blood-brain barrier.

### Example 10. Verification of the effects of different polypeptides on morphine-induced CPP in mice

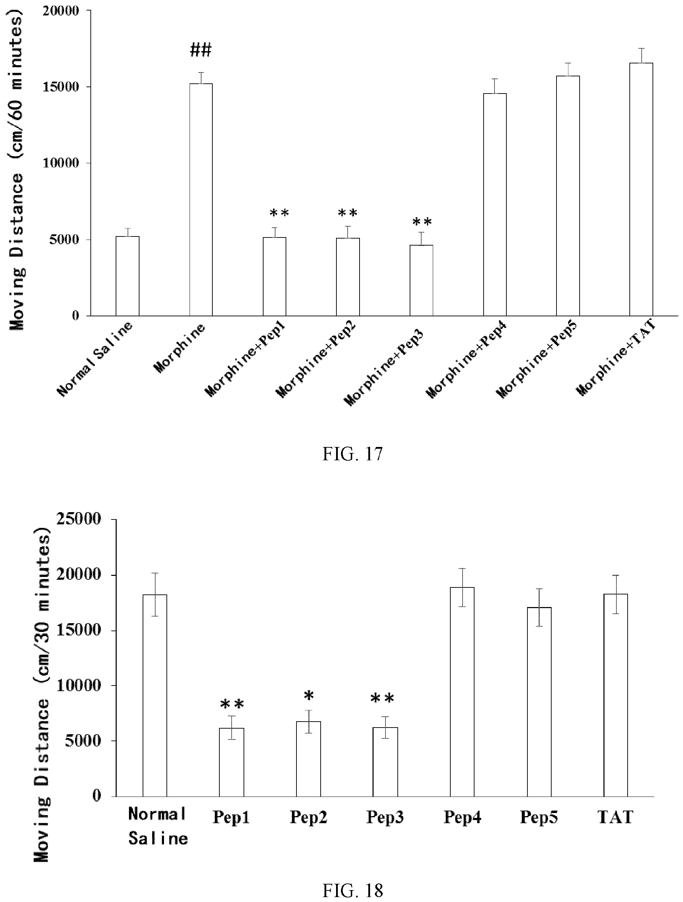

Mice were given morphine (1mg/kg) or normal saline for 8 days, and different polypeptides (3nmol/g) of Example 9 were administered to mice via intraperitoneal injection (10ml/kg) on Day 10; the expression of morphine-induced CPP was recorded according to the CPP experimental method in Example 5 on the second day after administration, and the results were shown in Fig. 17.

As can be seen from Fig. 17, Pep1 and truncated Pep2 and Pep3 significantly reduced the activity distance of morphine-CPP mice, while Pep4 and Pep5 which were substituted in the mid-segment failed to significantly reduce the activity distance, suggesting that the key motif of the polypeptide was located in the mid-segment of Pep1.

### Example 11. Verification of the effects of different polypeptides on calorie-induced BS in mice

In order to verify that the polypeptide of the invention is also effective against cocaine-induced addiction, the following tests were carried out:

Mice were given cocaine (15 mg/kg) or normal saline and then tested for 30 minutes, repeated for 5 days, and followed by a 5-day pause of administration. On the sixth day of the pause, 1 hour before cocaine administration, different polypeptides of Example 9 (3 nmol/g) were administered to mice via intraperitoneal injection (10 ml/kg) for drug induction, and a testing was carried out, and the results were shown in Fig. 18.

As can be seen from Fig. 18, as expected, Pep1 and truncated Pep2 and Pep3 significantly inhibited the expression of sensitization behavior, while Pep4 and Pep5 which were substituted in the mid-segment failed to inhibit the expression of sensitization behavior.

### Example 12. Verification of the effects of different polypeptides on alcohol-induced BS in mice

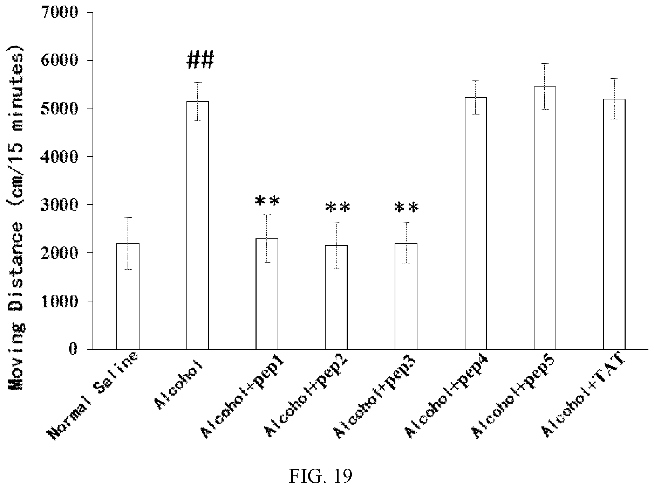

In order to verify that the polypeptide of the invention is also effective against alcohol-induced addiction, the following tests were carried out:
The training period lasted for 10 days, during which mice were administered alcohol (2.2g/kg) or normal saline every other day and then immediately tested for 15 minutes. On the third day after training, 1 hour before alcohol administration, different polypeptides of Example 9 (3 nmol/g) were administered to mice via intraperitoneal injection (10 ml/kg) for drug induction, and a testing was carried out, and the results were shown in Fig. 19.

As can be seen from Fig. 19, as expected, Pep1 and truncated Pep2 and Pep3 significantly inhibited the expression of sensitization behavior, while Pep4 and Pep5 which were substituted in the mid-segment failed to inhibit the expression of sensitization behavior.

## Claims

1. Use of a polypeptide in the preparation of a medicament for treating and/or preventing substance addiction, wherein the polypeptide consists of at least 11 consecutive amino acid residues of the sequence as set forth in SEQ ID NO: 1 and contains the sequence as set forth in SEQ ID NO: 2.

2. Use of a polypeptide in the preparation of a medicament for treating and/or preventing the relapse of substance addiction, wherein the polypeptide consists of at least 11 consecutive amino acid residues of the sequence as set forth in SEQ ID NO: 1 and contains the sequence as set forth in SEQ ID NO: 2.

3. The use of claim 2, wherein the relapse is caused by an environmental factor, an addictive substance factor or a stress factor.

4. The use according to any one of claims 1 to 3, wherein the amino acid sequence of the polypeptide is selected from the sequences as set forth in SEQ ID NO: 1, SEQ ID NO: 3 and SEQ ID NO: 4.

5. The use according to any one of claims 1 to 4, wherein the addiction is induced by a substance selected from one or more of the group consisting of morphine, nicotine, alcohol, cocaine, codeine, dihydrocodeine, hydromorphone, oxycodone, methadone, morphine, fentanyl, and pethidine.

6. Use of a nucleic acid molecule in the preparation of a medicament for treating and/or preventing substance addiction or the relapse of substance addiction, wherein the nucleic acid molecule encodes the polypeptide of any one of claims 1 to 5.

7. Use of an expression vector in the preparation of a medicament for treating and/or preventing substance addiction or the relapse of substance addiction, wherein the expression vector comprises the nucleic acid molecule of claim 6.

8. Use of a host cell in the preparation of a medicament for treating and/or preventing substance addiction or the relapse of substance addiction, wherein the host cell comprises the nucleic acid molecule of claim 6 or the expression vector of claim 7.

9. A complex comprising the polypeptide of any one of claims 1 to 5 and a carrier linked thereto for permeating the blood-brain barrier, alternatively the carrier for permeating the blood-brain barrier is selected from the group consisting of: HIV-1 Tat protein, insulin, cationized albumin, monoclonal antibody against rat transferrin receptor, mouse-derived monoclonal antibody against human insulin receptor, Penetratin, transduction domain of Tat protein, Pep-1 peptide, S4₁₃-PV, Magainin 2 and Buforin 2.

10. A polypeptide consisting of at least 11 and at most 14 consecutive amino acid residues of the sequence as set forth in SEQ ID NO: 1 and containing the sequence as set forth in SEQ ID NO: 2.
